# EUROPEAN PATENT APPLICATION

(11) **EP 4 154 873 A1**
(43) Date of publication of application: **29.03.2023**
(21) Application number: 22196568.4
(22) Date of filing: 20.09.2022
(51) Int. Cl.: A61K 9/20, A61K 31/506, A61P 9/12

(54) **THE TABLET COMPRISING MACITENTAN**

(30) Priority: 22.09.2021 TR 202114838
(71) Applicant: SANOVEL ILAC SANAYI VE TICARET A.S., Istanbul 34460 (TR)
(72) Inventor: ERDOGAN, Akif, Istanbul (TR); KARABULUT, Serif, Istanbul (TR); BILGEHAN ATAK, Fadime, Istanbul (TR); SUNEL, Fatih, Istanbul (TR)
(74) Representative: Sevinç, Erkan

(57) **Abstract**

The present invention relates to a tablet comprising macitentan or a pharmaceutically acceptable salt and at least one disintegrant and at least one the pharmaceutically acceptable excipients wherein disintegrants are selected from the group comprising croscarmellose sodium, crospovidone, carboxymethyl cellulose, poloxamer or mixtures thereof. The present invention also relates to a simple, rapid, cost effective, time-saving and industrially convenient method of preparing the tablet.

## Description

### Field of the Invention

The present invention relates to a tablet comprising macitentan or a pharmaceutically acceptable salt and at least one disintegrant and at least one the pharmaceutically acceptable excipients wherein disintegrants are selected from the group comprising croscarmellose sodium, crospovidone, carboxymethyl cellulose, poloxamer or mixtures thereof. The present invention also relates to a simple, rapid, cost effective, time-saving and industrially convenient method of preparing the tablet.

### Background of the Invention

Pulmonary arterial hypertension (PAH) is a disease that accumulates pulmonary artery endothelial cells, muscle layer and adventitia, which restricts pulmonary artery blood flow, which leads to increased pulmonary vascular resistance, progressive increase in pulmonary artery pressure, and finally causes the right heart A malignant cardiovascular disease of failure or even death. Its main feature is the gradual increase in pulmonary vascular resistance (PVR) and pulmonary arterial pressure (PAP) caused by pulmonary artery obstruction, accompanied by irreversible pulmonary vascular remodeling. Patients with severe symptoms can cause right heart failure and death, with poor prognosis and high mortality.

Macitentan is an endothelin receptor antagonist, which prevents endothelin (ET-1) from binding to ETA and ETB receptors, and exhibits high affinity and sustained occupancy in human pulmonary artery smooth muscle cells.

The chemical name of macitentan is N-[5-(4-Bromophenyl)-6-[2-[(5-bromo-2-pyrimidinyl)oxy]ethoxy]-4-pyrimidinyl]-N'-propylsulfamide and its chemical structure is shown in the Formula 1.

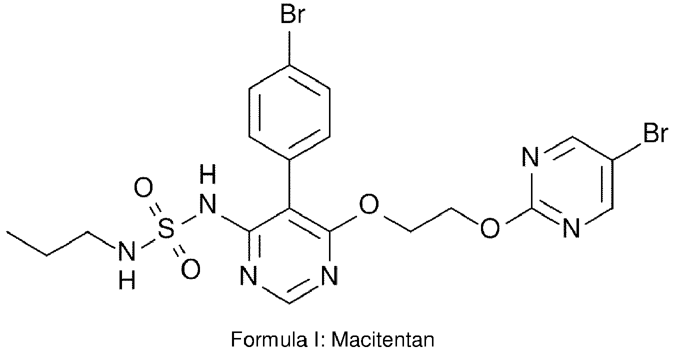

Macitentan is the active ingredient marketed under the brand OPSUMIT^{®} for the long-term treatment of pulmonary arterial hypertension.

Macitentan is a BCS class II drug substance characterized by low solubility and high permeability. The poor solubility and low dissolution rate of poorly water-soluble drugs in the aqueous gastrointestinal fluids often cause insufficient bioavailability. It is known that for BCS class II drugs rate limiting step is drug release from the dosage form and solubility in the gastric fluid and not the absorption, so increasing the solubility in turn increases the bioavailability for BCS class II drugs.

WO 2002053557 A1 application discloses macitentan and its preparation method.

WO 2007031933 A1 application discloses solid oral pharmaceutical compositions of macitentan. The results hint that, of all the possibilities tested, the compositions evidencing better behaviour were the ones comprising a high dosage of lactose or lactose monohydrate as filler.

In prior art, there are also several patents which disclose macitentan in tablet forms. However, despite the dissolution problem of macitentan, an effective formulation and method has not been disclosed.

There still remains a need in the art to provide an improved tablet composition of macitentan, having high solubility, dissolution rate, and accordingly a high bioavailability and a long-term stability which is also obtained by using an effective process.

### Detailed Description of the Invention

The main object of the present invention relates to a tablet composition comprising macitentan which eliminates the problems of dissolution and bioavailability and provides additional advantages to the relevant field of art.

Another object of the present invention is to provide a tablet composition which provides the desired stability.

Another object of the present invention is to provide a composition which is characterized by excellent pharmacotechnical properties, such as flowability, compressibility and content uniformity.

According to this embodiment of the present invention, a tablet composition comprises macitentan or a pharmaceutically acceptable salt and at least one disintegrant and at least one the pharmaceutically acceptable excipients wherein;
- disintegrants are selected from the group comprising croscarmellose sodium, crospovidone, carboxymethyl cellulose, poloxamer or mixtures thereof. Using said disintegrants provides adequate disintegrating properties for the tablets of the present invention but also it provides homogeneity of the active pharmaceutical ingredient which does not degrade to unwanted impurities.

According to this embodiment of the present invention, the disintegrant is croscarmellose sodium.

According to this embodiment of the present invention, the disintegrant is crospovidone.

According to this embodiment of the present invention, the amount of disintegrants is 0.1% to 20.0% by weight of the total core tablet.

According to this embodiment of the present invention, the amount of disintegrants is 1.0% to 10.0% or preferably 1.0% to 5.0% by weight of the total core tablet.

According to this embodiment of the present invention, the amount of macitentan or a pharmaceutically acceptable salt is 5.0% to 25.0% or preferably 10.0% to 20.0% by weight of the total core tablet.

According to this embodiment of the present invention, macitentan is present in the form of amorphous.

In general terms, excipients provided in a formulation may positively or negatively influence the physicochemical and pharmacokinetic properties, e.g. the solubility, absorption, bioavailability of an active agent. Macitentan may interact with several excipients. For this reason, the excipients which accompany an active agent have to be selected in a careful and conscious manner while a formulation) is developed. The formulations should have no physicochemical incompatibility between the active agent and the excipients.

According to this embodiment of the present invention, the pharmaceutically acceptable excipients are selected from the group comprising fillers, binders, surfactants, lubricants or mixtures thereof.

Suitable fillers are selected from group comprising lactose monohydrate, microcrystalline cellulose, mannitol, lactose, pregelatinized starch, ammonium alginate, calcium carbonate, anhydrous lactose, calcium phosphate, calcium phosphate dehydrate, neutral pellets, calcium sulfate, cellulose, cellulose acetate, fructose, glyceryl palmitostearate, kaolin, lactitol, magnesium carbonate, magnesium oxide, maltodextrin, maltose, medium chain triglycerides, polydextrose, polymethacrylates, sodium alginate, sodium chloride, sorbitol, starch, sucrose, sugar sphericals, sulfobutylether beta-cyclodextrin, talc, tragacanth, trehalose, xylitol or mixtures thereof.

According to this embodiment of the present invention, the filler is lactose monohydrate or lactose or microcrystalline cellulose or mixtures thereof.

According to this embodiment of the present invention, the filler is lactose monohydrate and microcrystalline cellulose.

According to one embodiment, lactose monohydrate is a filler which has the good flowability properties among the other fillers. In this invention, it further improves the disintegration of compositon as well as being a filler. In addition, it further enhances the compressibility by increasing the hardness of the tablet.

According to this embodiment of the present invention, the amount of filler is 55.0% to 90.0% by weight of the total core tablet.

According to this embodiment of the present invention, the amount of filler is 70.0% to 85.0% by weight of the total core tablet.

Suitable binders are selected from the group comprising polyvinylpyrrolidone, hydroxypropyl methylcellulose, croscarmellose sodium, cellulose acetate phthalate, starch, ethylcellulose, glyceryl behenate, hydroxyethyl cellulose, hydroxyethylmethyl cellulose, hydroxypropyl cellulose, hydroxypropyl starch, magnesium aluminum silicate, maltodextrin, methylcellulose, polydextrose, polyethylene oxide, polymethacrylates, aluminia hydroxide, stearic acid, sucrose, polyoxyethilene-alkyl ethers, pullulan or mixtures thereof.

According to this embodiment of the present invention, the binder is polyvinylpyrrolidone or hydroxypropyl methylcellulose or mixtures thereof.

According to this embodiment of the present invention, the binder is polyvinylpyrrolidone.

According to this embodiment of the present invention, the binder is hydroxypropyl methylcellulose.

According to this embodiment of the present invention, the amount of binder is 0.1% to 15.0% by weight of the total core tablet.

According to this embodiment of the present invention, the amount of binder is 1.0% to 10.0%, preferably 1.0% to 7.0% by weight of the total core tablet.

A surfactant is a substance that is generally a compound which lowers the surface tension between two liquids or between a liquid and a solid. Suitable surfactants are selected from the group comprising polysorbates, sodium lauryl sulphate, benzalconium chloride, docusate sodium, glyceryl esters, glyceryl monoleate, poloxamer, polyethylene alkyl ethers, polyglyceryl esters, polyoxyetylene esters, polyoxyetylene stearates, sodium stearate, hexadecyl pyridinium chloride or mixtures thereof.

According to this embodiment of the present invention, the surfactant is polysorbates.

According to this embodiment of the present invention, the amount of surfactant is 0.05% to 2.0% by weight of the total core tablet.

A polysorbate included in a tablet according to the present invention will have a mean polymerisation degree of from 20 to 100 monomer units, and may in the present invention be polysorbate 80.

According to this embodiment of the present invention, polysorbate 80 at least one binder is dissolved in a solvent. Then, the solution comprising polysorbate 80 is mixed other excipients and active agent. This provides both the desired content uniformity and the desired dissolution profile. Also, this helps to provide the desired flowability and compressibility.

Suitable lubricants are selected from the group comprising magnesium stearate, sodium stearyl fumarate, calcium stearate, zinc stearate, talc, sodium chlorate, magnesium lauryl sulfate, sodium oleate, sodium acetate, sodium benzoate, polyethylene glycol, stearic acid, fatty acid, fumaric acid, glyceryl palmito sulphate or mixtures thereof.

According to this embodiment of the present invention, the lubricant is magnesium stearate or sodium stearyl fumarate.

According to this embodiment of the present invention, the lubricant is magnesium stearate.

According to this embodiment of the present invention, the lubricant is sodium stearyl fumarate.

According to this embodiment of the present invention, the amount of lubricant is 0.1% to 5.0% by weight of the total core tablet.

According to one embodiment of the present invention, the tablet is coated with film coating.

Suitable film coating agent are selected from the group comprising polyvinyl alcohol (PVA), talc, titanium dioxide, glycerol, sodium lauryl sulphate, GMCC Type 1 (Glycerol monocaprylocaprate Type I) / Glycerol ester, polymethacrylates, hydroxypropyl methylcellulose, lactose monohydrate, hydroxypropyl cellulose, polyethylene glycol (PEG), talc, polyvinyl alcohol-polyethylene glycol copolymers (Kollicoal^{®} IR), ethylcellulose dispersions (Surelease^{®}), polyvinylpyrrolidone, polyvinylprolidone-vinyl acetate copolymer (PVP-VA), all kinds of Opadry^{®}, pigments, dyes, iron oxide or mixtures thereof or mixtures thereof.

According to one embodiment of the present invention, the film coating agent are polyvinyl alcohol, talc, titanium dioxide, glycerol, sodium lauryl sulphate.

According to one embodiment of the present invention, the tablet is obtained by using a wet granulation method therefore, a simple and low-cost production method was employed. Wet granulation process efficiently counteracts segregation, so it can achieve good dissolution and disintegration properties. Also, the process provides a desired uniformity of content. Solvents are used in the process.

The solvent is selected from the group comprising water, acetone, ethanol, isopropanol or mixtures thereof. Preferably, water is used in the invention.

According to this embodiment of the present invention, the active ingredient is found in a low amount in the core tablet as a percentage. In the wet process, a binder solution is prepared with at least one surfactant and at least one binder and a solvent, then mixing with the mixture comprising active agents. When the film-coated tablet is prepared by the above-mentioned process it was seen that it does not lead to losses in the active agent or excipients during production and provides desired good content uniformity. It reflects that content uniformity play important role in the dissolution of the drug.

According to this embodiment of the present invention, a binder solution comprises polyvinylpyrrolidone and polisorbate (80).

According to this embodiment of the present invention, the tablet has a hardness between 50N to 70N, preferably between 55 N to 65N, this provides high chemical and mechanical stability, thus it is not brittle easily. The measurement is carried out on ERWEKA TBH125 in the present invention.

According to this embodiment of the present invention, a tablet comprises;
- Macitentan
- Lactose monohydrate
- Microcrystalline cellulose (PH 101)
- Croscarmellose sodium
- Polyvinylpyrrolidone or HPMC
- Polisorbate (80)
- Sodium stearyl fumarate or magnesium stearate.

According to this embodiment of the present invention, a tablet comprises;
- 5.0-25.0% by weight of Macitentan
- 45.0-65.0% by weight of Lactose monohydrate
- 11.0-35.0% by weight of Microcrystalline cellulose (PH 101)
- 0.1-20.0% by weight of Croscarmellose sodium
- 1.0-7.0% by weight of Polyvinylpyrrolidone or HPMC
- 0.05-2.0% by weight of Polisorbate (80)
- 0.1-5.0% by weight of Sodium stearyl fumarate or magnesium stearate.

According to this embodiment of the present invention, a tablet comprises;
- 11.0-16.0% by weight of Macitentan
- 45.0-65.0% by weight of Lactose monohydrate
- 11.0-35.0% by weight of Microcrystalline cellulose (PH 101)
- 1.0-5.0% by weight of Croscarmellose sodium
- 1.0-7.0% by weight of Polyvinylpyrrolidone or HPMC
- 0.05-1.0% by weight of Polisorbate (80)
- 0.1-3.0% by weight of Sodium stearyl fumarate or magnesium stearate.

According to another embodiment of the present invention, a process for preparing tablet composition processed wet granulation comprises the following steps;
a) Dissolving polysorbate and at least one binder (polyvinylpyrrolidone or HPMC) in water in homogenizer,
b) Mixing macitentan, lactose monohydrate, microcrystalline cellulose (ph 101) and croscarmellose sodium,
c) Adding the prepared granulation suspension at step (a) into powder mixture at step (b) and mixing to obtained wet granules,
d) Drying and obtained dry granules,
e) Sieving the dry granules,
f) Adding croscarmellose sodium and microcrystalline cellulose then mixing,
g) Adding at least one lubricant (magnesium stearate or Sodium stearyl fumarate) and mixing,
h) Compressing to form of tablets,
i) Coating tablets with film coating.

### Example 1:

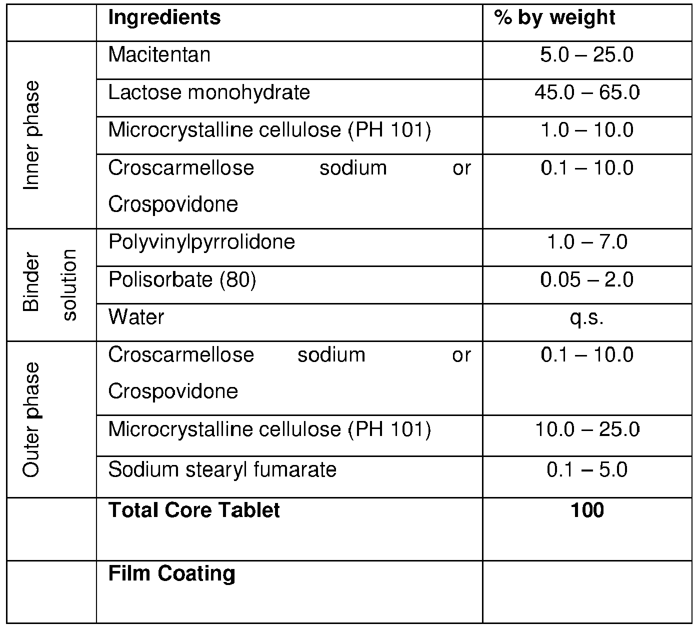

### Example 2:

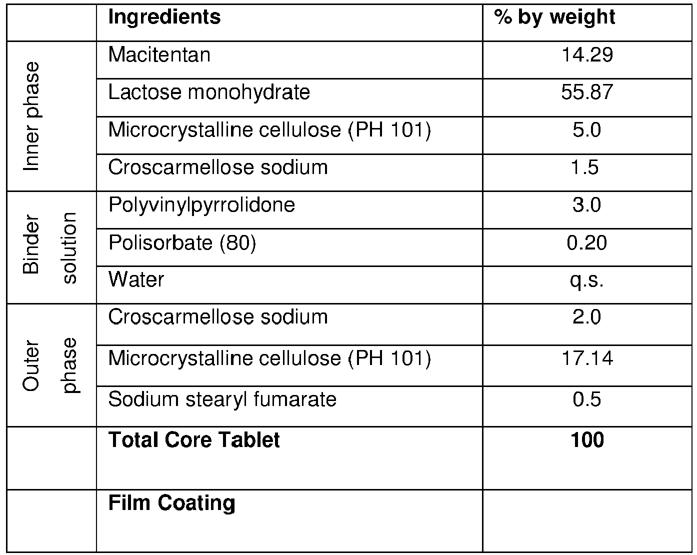

### Example 3;

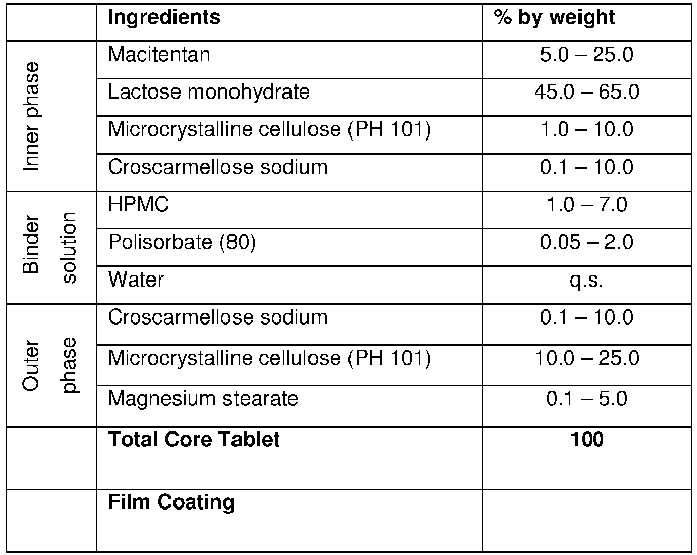

### Example 4:

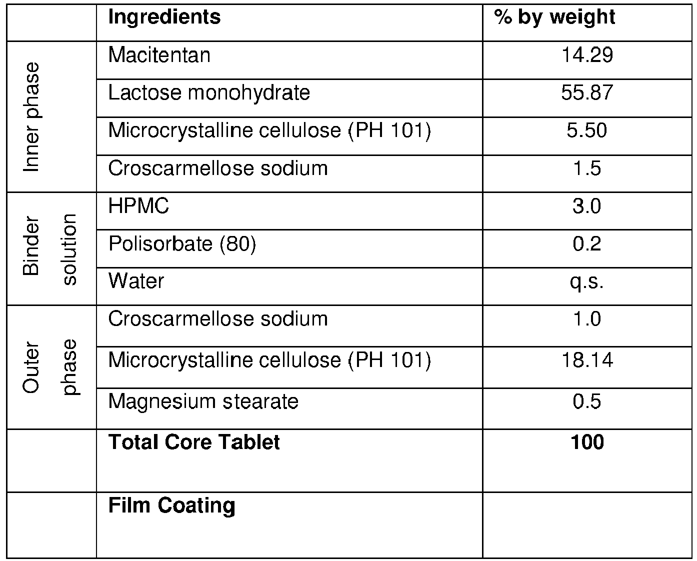

**A process for example 1 or 2 or 3 or 4;**
a) Dissolving polysorbate and at least one binder (polyvinylpyrrolidone or HPMC) in water in homogenizer,
b) Mixing macitentan, lactose monohydrate, microcrystalline cellulose (ph 101) and croscarmellose sodium,
c) Adding the prepared granulation suspension at step (a) into powder mixture at step (b) and mixing to obtained wet granules,
d) Drying and obtained dry granules,
e) Sieving the dry granules,
f) Adding croscarmellose sodium and microcrystalline cellulose then mixing,
g) Adding at least one lubricant (magnesium stearate or Sodium stearyl fumarate) and mixing,
h) Compressing to form of tablets,
i) Coating tablets with film coating.

Film coating comprises polyvinyl alcohol, talc, titanium dioxide, glycerol, sodium lauryl sulfate.

## Claims

1. A tablet composition comprising macitentan or a pharmaceutically acceptable salt and at least one disintegrant and at least one the pharmaceutically acceptable excipients wherein;
- disintegrants are selected from the group comprising croscarmellose sodium, crospovidone, carboxymethyl cellulose, poloxamer or mixtures thereof.

2. The tablet according to claim 1, wherein the disintegrant is croscarmellose sodium.

3. The tablet according to claim 1, wherein the amount of macitentan or a pharmaceutically acceptable salt is 5.0% to 25.0% or preferably 10.0% to 20.0% by weight of the total core tablet.

4. The tablet according to claim 1, wherein the pharmaceutically acceptable excipients are selected from the group comprising fillers, binders, surfactants, lubricants or mixtures thereof.

5. The tablet according to claim 4, wherein fillers are selected from group comprising lactose monohydrate, microcrystalline cellulose, mannitol, lactose, pregelatinized starch, ammonium alginate, calcium carbonate, anhydrous lactose, calcium phosphate, calcium phosphate dehydrate, neutral pellets, calcium sulfate, cellulose, cellulose acetate, fructose, glyceryl palmitostearate, kaolin, lactitol, magnesium carbonate, magnesium oxide, maltodextrin, maltose, medium chain triglycerides, polydextrose, polymethacrylates, sodium alginate, sodium chloride, sorbitol, starch, sucrose, sugar sphericals, sulfobutylether beta-cyclodextrin, talc, tragacanth, trehalose, xylitol or mixtures thereof.

6. The tablet according to claim 5, wherein the amount of filler is 55.0% to 90.0% by weight of the total core tablet.

7. The tablet according to claim 4, wherein binders are selected from the group comprising polyvinylpyrrolidone, hydroxypropyl methylcellulose, croscarmellose sodium, cellulose acetate phthalate, starch, ethylcellulose, glyceryl behenate, hydroxyethyl cellulose, hydroxyethylmethyl cellulose, hydroxypropyl cellulose, hydroxypropyl starch, magnesium aluminum silicate, maltodextrin, methylcellulose, polydextrose, polyethylene oxide, polymethacrylates, aluminia hydroxide, stearic acid, sucrose, polyoxyethilene-alkyl ethers, pullulan or mixtures thereof.

8. The tablet according to claim 7, wherein the binder is polyvinylpyrrolidone or hydroxypropyl methylcellulose or mixtures thereof.

9. The tablet according to claim 4, wherein lubricants are selected from the group comprising magnesium stearate, sodium stearyl fumarate, calcium stearate, zinc stearate, talc, sodium chlorate, magnesium lauryl sulfate, sodium oleate, sodium acetate, sodium benzoate, polyethylene glycol, stearic acid, fatty acid, fumaric acid, glyceryl palmito sulphate or mixtures thereof.

10. The tablet according to claim 9, wherein the lubricant is magnesium stearate or sodium stearyl fumarate.

11. The tablet according to claim 1, wherein the tablet has a hardness between 50N to 70N, preferably between 55 N to 65N.

12. The tablet according to claim 1, wherein the tablet comprising;
- Macitentan
- Lactose monohydrate
- Microcrystalline cellulose (PH 101)
- Croscarmellose sodium
- Polyvinylpyrrolidone or HPMC
- Polisorbate (80)
- Sodium stearyl fumarate or magnesium stearate.

13. The tablet according to claim 1, wherein comprising
- 5.0-25.0% by weight of Macitentan
- 45.0-65.0% by weight of Lactose monohydrate
- 11.0-35.0% by weight of Microcrystalline cellulose (PH 101)
- 0.1-20.0% by weight of Croscarmellose sodium
- 1.0-7.0% by weight of Polyvinylpyrrolidone or HPMC
- 0.05-2.0% by weight of Polisorbate (80)
- 0.1-5.0% by weight of Sodium stearyl fumarate or magnesium stearate.

14. The tablet according to claim 1, wherein comprising
- 11.0-16.0% by weight of Macitentan
- 45.0-65.0% by weight of Lactose monohydrate
- 11.0-35.0% by weight of Microcrystalline cellulose (PH 101)
- 1.0-5.0% by weight of Croscarmellose sodium
- 1.0-7.0% by weight of Polyvinylpyrrolidone or HPMC
- 0.05-1.0% by weight of Polisorbate (80)
- 0.1-3.0% by weight of Sodium stearyl fumarate or magnesium stearate.

15. A process for preparing tablet composition processed wet granulation comprising the following steps;
a) Dissolving polysorbate and at least one binder (polyvinylpyrrolidone or HPMC) in water in homogenizer,
b) Mixing macitentan, lactose monohydrate, microcrystalline cellulose and croscarmellose sodium,
c) Adding the prepared granulation suspension at step (a) into powder mixture at step (b) and mixing to obtained wet granules,
d) Drying and obtained dry granules,
e) Sieving the dry granules,
f) Adding croscarmellose sodium and microcrystalline cellulose then mixing,
g) Adding at least one lubricant (magnesium stearate or Sodium stearyl fumarate) and mixing,
h) Compressing to form of tablets,
i) Coating tablets with film coating.
